# FASCICULE DE BREVET EUROPEEN

(11) **EP 4 574 054 B1**
(45) Date de publication et mention de la délivrance du brevet: **19.08.2026**
(21) Numéro de dépôt: 24218567.6
(22) Date de dépôt: 10.12.2024
(51) Int. Cl.: A61B 8/08

(54) **METHODE DE CARACTERISATION D'UN OBJET DE FORME TUBULAIRE TEL QU'UN VAISSEAU SANGUIN PAR IMAGERIE ULTRASONORE**
VERFAHREN ZUR CHARAKTERISIERUNG EINES ROHRFÖRMIGEN OBJEKTS WIE ETWA EINES BLUTGEFÄSSES MITTELS ULTRASCHALLBILDGEBUNG
METHOD FOR CHARACTERIZING A TUBULAR OBJECT SUCH AS A BLOOD VESSEL BY ULTRASOUND IMAGING

(30) Priorité: 18.12.2023 FR 2314398
(43) Date de publication de la demande: 25.06.2025
(73) Titulaire: Commissariat à l'Energie Atomique et aux Energies Alternatives, 75015 Paris (FR)
(72) Inventeur: CHATILLON, Sylvain, 91191 GIF-SUR-YVETTE Cedex (FR); BELHADJ, Djallel, 91191 GIF-SUR-YVETTE Cedex (FR)
(74) Mandataire: Atout PI Laplace

(56) Documents cités:
- FLEAGLE S R ET AL: "AUTOMATED ANALYSIS OF CORONARY ARTERIAL MORPHOLOGY IN CINEANGIOGRAMS: GEOMETRIC AND PYSIOLOGIC VALIDATION IN HUMANS", IEEE TRANSACTIONS ON MEDICAL IMAGING, IEEE, USA, vol. 8, no. 4, 1 December 1989 (1989-12-01), pages 387 - 400, XP000086315, ISSN: 0278-0062, DOI: 10.1109/42.41492
- HENNERSPERGER CHRISTOPH ET AL: "Multi-Scale Tubular Structure Detection in Ultrasound Imaging", IEEE TRANSACTIONS ON MEDICAL IMAGING, IEEE, USA, vol. 34, no. 1, 1 January 2015 (2015-01-01), pages 13 - 26, XP011568808, ISSN: 0278-0062, [retrieved on 20141224], DOI: 10.1109/TMI.2014.2340912

## Description

L'invention concerne le domaine de l'imagerie ultrasonore, en particulier l'imagerie médicale et porte plus précisément sur une méthode permettant de déterminer le centre et le diamètre d'un objet de forme tubulaire tel qu'un vaisseau sanguin.

L'imagerie ultrasonore, en particulier dans le domaine médical, vise à imager différents types d'objets ou d'organes du corps humain pour mieux les caractériser.

Dans le domaine de l'imagerie médicale, il existe un besoin pour caractériser les vaisseaux sanguins, en particulier les artères. En effet, les artères sont un véritable indicateur de santé, car elles donnent des informations essentielles sur l'état de santé du corps humain. Le monitoring temps réel des artères permet le suivi de marqueurs biologiques tels que la pression artérielle pour mieux comprendre le fonctionnement du corps humain et anticiper le développement de maladies. Le développement des technologies à base d'ultrasons permet de concevoir des systèmes d'imagerie compacts qui offrent une solution non invasive. Le monitoring temps réel exige que toute la chaîne de mesure, allant de l'acquisition, la localisation de l'artère, jusqu'à l'extraction d'informations pertinentes, nécessite d'être entièrement automatisée.

Dans ce domaine général, il existe un besoin particulier pour caractériser les artères via la détection de leur centre et la mesure de leur diamètre.

Les méthodes d'imagerie ultrasonore qui proposent des solutions pour caractériser des vaisseaux sanguins sont le plus souvent focalisées sur la dimension longitudinale du vaisseau, par exemple de l'artère carotide.

Ces méthodes sont diverses mais on peut les grouper en trois catégories non exhaustives qui comprennent des méthodes manuelles, semi-automatiques et automatiques.

Les méthodes manuelles se basent sur l'expertise humaine pour déterminer manuellement la position d'artères, par exemple à l'aide d'outils graphiques appliqués sur des images échographiques.

Dans le cas des méthodes semi-automatiques, des algorithmes d'assistance fonctionnent en collaboration avec un humain. Par exemple, l'algorithme peut continuer à localiser automatiquement l'artère en se basant sur l'humain comme un guide, qui valide, corrige, et indique une information de référence aidant l'algorithme à trouver la bonne localisation.

Dans la catégorie des méthodes totalement automatiques, l'algorithme détermine automatiquement la localisation d'artère. La plupart de ces méthodes automatiques traitent spécifiquement le problème de localisation de l'artère carotide et ne fonctionnent pas de façon optimale avec d'autres artères. En effet, le cas de l'artère carotide est un cas moins contraignant que d'autres artères telles que l'artère radiale pour plusieurs raisons. L'artère carotide est plus grande avec des parois plus épaisses et présente un profil d'intensité différent de celui de l'artère radiale, avec moins de bruit, et plus de contraste. Le diamètre de la carotide étant plus important que celui de l'artère radiale, les images échographiques présentent un meilleur contraste. Il est ainsi plus facile de repérer le centre de l'artère carotide rien qu'en recherchant la zone avec le profil d'intensité le plus faible. En comparaison avec l'artère radiale, les images ultrasonores de la carotide présentent naturellement moins d'objets anatomiques qui peuvent être confondus avec l'artère. La plus grande taille de l'artère carotide contribue aussi, car elle permet de limiter les champs des images ultrasonores ce qui permet d'obtenir des images qui contiennent le moins d'objets bruyants. La plupart des méthodes développées pour l'artère carotide focalisent sur la dimension longitudinale, ce qui permet de faciliter d'avantage la localisation en repérant simplement la trace horizontale au profil d'intensité le plus bas. Les autres objets anatomiques ont une empreinte moins bruyante sur cette dimension. La plupart des algorithmes localisent verticalement la zone qui a l'intensité la plus faible, en balayant toute l'image, par la suite, l'artère est localisée selon un consensus des points repérés après suppression des valeurs aberrantes. Ces méthodes basées sur la dimension longitudinale sont sensibles à l'orientation de la sonde et nécessitent, toutes, un travail de positionnement précis de la sonde sur la peau lors de l'acquisition. Une incertitude d'orientation rendra la mesure du diamètre et d'autres propriétés peu précises. Il est possible de contourner ce problème en utilisant des sondes multidimensionnelles mais celles-ci sont plus coûteuses.

Bien que la dimension transversale soit plus difficile à traiter, car la forme des objets anatomiques sur cette dimension apparait frontalement et perturbe la localisation notamment dans le cas de l'artère radiale, l'avantage de la dimension transversale est qu'elle ne nécessite pas un positionnement précis de la sonde sur la peau, et les mesures de diamètre restent robustes et fiables vis-à-vis des erreurs d'orientation de la sonde.

Les solutions existantes de localisation automatique sur la dimension transversale des artères ou plus généralement des vaisseaux sanguins sont rares et souffrent toutes d'une ou plusieurs limitations. Les méthodes basées sur l'apprentissage profond sont efficaces mais consomment beaucoup d'énergie, ne sont pas adaptées aux systèmes portables de petite taille, elles sont complexes de mise en œuvre et exigent énormément de ressources. Il est difficile d'atteindre des performances temps réel notamment lorsqu'il s'agit d'un calculateur portable. On trouve ces méthodes intégrées dans les interfaces d'assistance pour lesquelles les exigences ne sont pas limitées en temps et en ressources. De même, les réseaux de neurones caractérisés par leur aspect boite noire, souffrent du problème d'imprédictibilité. Il a été démontré qu'un seul pixel d'une image peut conduire un réseau de neurones à fournir des données aberrantes. Ce problème est d'ailleurs la principale cause ralentissant l'adoption de l'intelligence artificielle dans le domaine médical, et les domaines à conséquences graves.

Les solutions basées sur le Doppler permettent une localisation grâce au changement de fréquence lié au flux sanguin. Ces méthodes sont limitées à la localisation verticale, où la profondeur de l'artère est repérée. La localisation horizontale est effectuée manuellement. Un autre inconvénient de ces méthodes est que la localisation par Doppler nécessite le monitoring des fréquences ultrasonores dans le temps, cela implique que toutes ces méthodes ont besoin de la dimension temporelle afin de localiser l'artère.

D'autres limitations se manifestent dans plusieurs aspects tels que par exemple, l'aspect rapidité d'exécution et la satisfaction des normes temps réel. Les algorithmes de recherche itératifs par exemple sont lents et nécessitent des ressources considérables pour atteindre un fonctionnement en temps réel.

Les algorithmes nécessitant un modèle de référence et des informations préalables sont handicapants. Un autre aspect est lié aux algorithmes qui sont sensibles au bruit et nécessitent un prétraitement pour conditionner les données reçues avant d'exécuter la méthode de localisation. Certaines solutions existantes se basent sur des points de repère tels que les contours, ce qui les rend vulnérables aux imperfections des données. Ces méthodes ont souvent recours à des algorithmes de prétraitement, de consensus et de suppression de valeurs aberrantes afin d'assurer la robustesse, ce qui ajoute en complexité et en calcul.

D'autres méthodes sont appliquées sur d'autres solutions technologiques qui sortent du cadre des ultrasons telles que les méthodes dites par ultrasons intravasculaires ou IVUS « Intravascular ultrasound » et les méthodes dites de tomographie par cohérence optique ou OCT « optical coherence tomographie ». Bien que ces méthodes traitent des images d'artères, elles ne traitent pas le même problème, car le profil des données traitées est différent, en terme d'intensité, de bruit et de difficultés. Aussi ces méthodes héritent des inconvénients de ces technologies telles que l'invasivité dans le cas des méthodes IVUS et la limitation de profondeur dans le cas des méthodes OCT non invasives.

La demande de brevet WO2016057233 présente une méthode d'estimation d'une forme d'onde de pression artérielle continue non invasive à l'aide d'ultrasons et d'un brassard automatisé. Elle appartient à la catégorie des mesures continues non invasives de la pression artérielle. Le système proposé mesure des caractéristiques physiques telles que la géométrie, l'élasticité et la déformation d'un vaisseau sanguin, ainsi que d'autres paramètres physiques externes. La modélisation informatique et le traitement des signaux mesurés sont utilisés pendant le gonflage et/ou le dégonflage du brassard pour estimer de manière itérative la pression artérielle du sujet. La partie ultrasonore permet d'estimer le diamètre de l'artère et de calculer son élasticité. L'article par Fleagle et al. "Automated analysis of coronary arterial morphology in cineangiograms: geometric and physiologic validation in humans", IEEE TRANSACTIONS ON MEDICAL IMAGING, 24 décembre 2014, décrit des méthodes de détection automatisée de structures tubulaires à l'aide de filtres spécialement adaptés à la modalité d'imagerie ultrasonore.

La localisation de l'artère dans cette solution est limitée par plusieurs points : d'abord, les réseaux de transducteurs sont fixés par un opérateur qualifié à un emplacement approximatif et l'artère est localisée en trois dimensions par une méthode de recherche itérative des caractéristiques géométriques et de la réponse ultrasonore supposées. La recherche est non seulement itérative, ce qui est gourmand en calcul, mais elle se fait dans un espace de recherche en 3D, donc sur une grande quantité de données. Cela limite la rapidité d'exécution notamment pour les appareils limités en ressource et en consommation énergétiques. De plus, cette recherche dans l'espace 3D est effectuée en comparant à chaque itération, avec un modèle d'artère de référence construit à travers des informations préalables personnelles au sujet, telles que son âge, sa taille, etc. Outre le fait que cette solution nécessite un modèle de référence et des informations préalables, elle nécessite aussi une sonde à deux dimensions pour permettre une acquisition dans l'espace 3D sur lequel s'exécute l'algorithme itératif de recherche. Avec une localisation lente, le rafraichissement de la position de l'artère impose un compromis pour la rapidité, la précision et le mouvement de la personne.

Le document [1] présente une méthode destinée à caractériser l'artère carotide qui peut s'appliquer sur la dimension longitudinale et transversale de cette artère. Elle consiste en un algorithme de détection de contours basé sur la méthode « Snake » et doté d'un modèle de référence d'artère mathématique sous formes de B-splines qui se déforme en échelle, en translation et en rotation. La méthode converge vers les contours d''artère carotide grâce à un algorithme d'optimisation. Le critère de convergence est assuré par une somme des différences absolues entre le modèle et l'image en chaque point de l'image. Les paramètres du modèle sont optimisés itérativement grâce à l'algorithme de minimisation de l'énergie, puis la somme des différences absolues est calculée en chaque point. La forme circulaire du modèle de référence est utilisée pour simplifier le calcul. Un algorithme d'optimisation à base de gradient est appliqué pour affiner une segmentation précise des contours d'artère détectés.

Dans ce document, la forme de l'artère carotide est assez grande et circulaire, et l'épaisseur de sa paroi est assez grande, ce qui permet de distinguer facilement les contours des couches, sur lequel l'algorithme se base. Cette solution n'est pas applicable à d'autres types d'artères telles que l'artère radiale pour plusieurs raisons. Tout d'abord l'artère radiale apparait souvent avec une forme ni parfaitement circulaire, ni parfaitement rectangulaire, car sa taille est petite. La paroi de l'artère radiale est petite, et ne permet pas de distinguer ses couches avec des sondes standards à faible fréquence centrale, cela nécessite des sondes de haute qualité à des fréquences ultrasonores plus élevées. Pour cette raison et vu que la méthode de la référence [1] s'appuie sur les différences de contraste local entre les contours intérieurs et extérieurs, il sera difficile pour l'algorithme de localiser avec précision la région d'intérêt dans les cas où l'artère ou le tissu environnant manque de texture ou de contraste, ce qui est le cas de l'artère radiale.

Aussi, les objets anatomiques sur les images transversales de la carotide tels que la veine jugulaire, ont des formes peu circulaires et facilement discriminables par rapport à l'artère carotide. Pour cette raison, l'algorithme ne risque pas de converger vers d'autres objets. Dans le cas de l'artère radiale, plusieurs objets anatomiques ont une forme et un positionnement très ressemblant à l'artère radiale, ce qui fait que la méthode risque de converger vers les autres objets au lieu de l'artère radiale, car, le critère de la somme des différences absolues sera satisfait aussi par exemple par les veines voisines de l'artère.

La méthode nécessite un prétraitement d'amélioration d'images à base d'histogrammes adaptatifs afin de réduire le bruit. Car le bruit risque d'augmenter considérablement le temps nécessaire de convergence des algorithmes d'optimisation sur lequel se base la méthode. Le temps de convergence est variable et dépend des profils d'intensité de l'image acquise, de sa complexité, de la position de l'artère et de sa ressemblance vis-à-vis du modèle. Il dépend aussi des paramètres d'initialisation intégrés au modèle. Si l'image contient des structures anatomiques complexes ou si l'artère elle-même a des formes irrégulières, l'approche de comparaison avec le modèle de référence peut ne pas fonctionner correctement dans les délais.

Aussi, le modèle peut ne pas être suffisamment flexible pour gérer ces variations, ce qui entraîne un mauvais alignement. Pour cela, cette méthode est plus adaptée aux applications qui ne nécessitent pas des contraintes temps réel stricte, et une mesure régulière en temps de vol et à des intervalles fixes. La méthode compare les pixels de l'image avec un modèle à chaque point de l'image tout en calculant à chaque fois le critère de somme des différences absolue sur les pixels. Cette opération est intense en calcul, ce qui impose à la méthode le recours à des algorithmes d'optimisation pour réduire le temps de calcul. L'inconvénient de cette technique est qu'elle rend la solution entièrement dépendante du temps de convergence de l'algorithme d'optimisation qui est un algorithme itératif dépendant de la complexité de l'image et vulnérable aux problèmes de convergence tels que les minimas locaux. De plus, l'optimisation peut converger vers les veines plutôt que l'artère sur les scans transversaux de l'artère radiale.

La méthode présentée dans le document [2] concerne la localisation de l'artère carotide dans des images transversales. La méthode est basée sur la transformée de Hough circulaire, une méthode qui permet de détecter les lignes dans l'image et qui a été adaptée pour la détection de cercles. La méthode procède au traitement des séquences de 5 à 15 images consécutives. Chaque image est prétraitée pour optimiser la luminosité et le contraste. Ensuite, un filtre Gaussien puissant réduit le bruit. Bien que plusieurs structures morphologiques fines aient été détruites dans ce processus, l'artère carotide apparait plus claire. Ensuite, la transformée de Hough détecte tous les cercles sombres en considérant tous les rayons qui se situent dans une plage raisonnable. Les coordonnées des centres détectés (xi, yi) et les rayons (ri) sont collectés. Pour chacun des cercles sombres détectés, les valeurs de luminosité de leurs pixels sont évaluées. Le cercle présentant les valeurs les plus sombres, c'est-à-dire la luminosité minimale, est élu comme "cercle candidat", et ses coordonnées de centre (xci, yci) avec un rayon (rci) sont enregistrés dans une matrice de candidats potentiels. Une fois que toutes les images de B-mode ont été traitées, la matrice contient les résultats obtenus à partir de chaque image. Le triplet (xc, yc, rc) le plus fréquent apparaissant dans la matrice est sélectionné comme choix final.

Cette troisième méthode n'est pas non plus adaptée à l'artère radiale pour les raisons suivantes. L'artère carotide a une forme assez circulaire, elle se distingue comme une zone assez noire et facile à discriminer par rapport aux autres objets anatomiques. La forme de l'artère radiale sur les images ultrasonores n'apparait pas parfaitement circulaire. De plus, il existe plusieurs objets anatomiques similaires à l'artère, par exemple les veines, qui sont voisines, parfois plus sombres que l'artère radiale et elles aussi avec une forme circulaire. La transformée de Hough peut être adaptée à d'autres formes plus complexes pour l'appliquer sur l'artère radiale, par ailleurs, cela augmente considérablement l'intensité des calculs, car la forme circulaire telle que présentée dans le document [2] effectue déjà une recherche dans un espace 3D ce qui nécessite un calcul beaucoup plus intense qu'avec la transformée de Hough standard. En effet, pour chaque diamètre de cercle, la méthode effectue le calcul d'un nouvel accumulateur sur toute l'image. L'artère radiale peut se déformer facilement, par exemple vers une forme d'ellipse ce qui implique un paramètre additionnel à prendre en considération par la transformée de Hough. La forme de l'artère radiale est beaucoup plus déformée que celle de l'artère carotide, car la taille est plus petite ce qui fait que les contours sont plus petits et donc plus déformés par le bruit observé sur les images ultrasonores. La transformée de Hough fournit des détections multiples et le choix selon la zone la plus sombre ne fonctionnera pas dans le cas de l'artère radiale, car les veines voisines peuvent être plus sombres. Ces détections multiples provoquent un grand nombre de fausses détections, ce qui impose soit l'utilisation de critères plus complexes, soit de considérer la dimension temporelle afin d'améliorer la robustesse de localisation. Pour cette raison, la méthode nécessite 5 images successives. La transformée de Hough circulaire nécessite une étape de détection de contours et des réglages de seuillages. La détection de contours est vulnérable au bruit dans l'image, ce qui implique la nécessité d'un prétraitement d'amélioration et de filtrage de bruit. Pour cette raison, la méthode dans le document, précise qu'un filtrage Gaussien puissant est nécessaire.

Toutes les méthodes précitées présentent les inconvénients décrits. Il existe alors un besoin pour une nouvelle méthode qui soit applicable à la caractérisation de la section transversale d'une artère en particulier de l'artère radiale qui ne nécessite pas de sonde bidimensionnelle et qui soit adaptée à l'environnement d'acquisition de l'artère à caractériser, notamment le bruit et la présence d'autres objets biologiques.

L'invention permet une localisation et une caractérisation automatique d'une artère radiale dans sa section transversale. Elle implique une première phase de localisation du centre de l'artère à partir de plusieurs acquisitions ultrasonores formant une image 2D de la section transversale comprenant la section de l'artère, puis une seconde phase de détermination du diamètre de l'artère à partir de la mesure ultrasonore correspondant au trajet passant par le centre de l'artère.

L'invention ne nécessite pas un positionnement précis de la sonde sur la peau par un expert, et tolère les incertitudes de positionnement angulaire. Cela permet d'avoir des mesures fiables même en présence du mouvement du sujet et de la sonde par rapport au sujet. La mesure du diamètre se fait au point horizontal le plus pertinent au centre de l'artère, qui est le point qui fournit la mesure la plus précise du diamètre.

Le principe de l'invention est basé sur une approche de filtre de convolution et comporte un filtre de base spécialement dédié pour la détection d'une artère ou plus généralement d'un objet de forme tubulaire. Une fois le centre de l'artère localisé, des méthodes pour extraire les propriétés de l'artère sont appliquées localement sur le résultat du filtre.

La méthode proposée a une faible complexité et peut être implémentée facilement, car la quasi-totalité des opérations de localisation est effectuée sous forme d'opérations simples, ce qui constitue un avantage de simplicité considérable et permet à la méthode d'être embarquée sur des systèmes portables à ressources limitées et d'effectuer des mesures en temps de vol. La méthode proposée ne nécessite aucune comparaison avec un modèle de référence, ni calcul d'un critère de ressemblance à chaque point, ni aucun calcul itératif intensif, ni de risque de convergence itérative et de problèmes locaux dont souffrent les algorithmes d'optimisation. Un autre avantage de l'invention, est qu'elle peut s'exécuter en synchronisation avec l'acquisition de façon incrémentale au fur et à mesure de la réception des signaux, sans avoir besoin d'attendre la fin de l'acquisition. Cela permet un rafraichissement rapide de la localisation de l'artère. Contrairement aux méthodes existantes, la méthode proposée n'a pas besoin de stocker un grand nombre de paramètres, car elle effectue graduellement une mise à jour de la sommation et des coordonnées du point extremum au fur et à mesure de l'évolution du calcul. Certaines solutions existantes se basent sur des points de repère tels que les contours, ce qui les rend vulnérables. Certaines de ces solutions ont recours à des méthodes d'amélioration du bruit ou de suppression de valeurs aberrantes, ce qui augmente la complexité et les calculs. La solution proposée se base sur l'information globale, ce qui la rend robuste envers le bruit et ne nécessite pas de prétraitement d'amélioration d'image, ni d'algorithmes de suppression de points aberrants.

La solution proposée effectue la localisation directement sur la dimension spatiale d'une seule acquisition transversale et n'a pas besoin d'acquisitions multiples sur la dimension temporelle à l'inverse notamment des méthodes basées sur le Doppler. La méthode proposée n'a pas besoin d'informations préalables sur la personne, ni d'un modèle d'artère de référence. Avec une localisation rapide, le rafraichissement de la position de l'artère se fait à grande vitesse et offre la possibilité d'avoir à la fois une fréquence de mesure plus élevée, plus de précision et plus de liberté de mouvement de la personne.

Bien que l'invention soit décrite dans le contexte particulier de la caractérisation d'un vaisseau sanguin pour des applications d'imagerie médicale, elle peut également s'appliquer dans le domaine du contrôle non destructif par ultrasons pour caractériser tout type d'objet de forme tubulaire.

L'invention a pour objet une méthode de caractérisation d'un objet de forme tubulaire par imagerie ultrasonore, la méthode comprenant les étapes de :
- Acquérir au moyen d'un transducteur ultrasonore, plusieurs signaux ultrasonores provenant de la réflexion d'un champ ultrasonore émis par le transducteur sur une zone d'intérêt dans un plan de coupe transversale de l'objet, pour différentes positions du transducteur par rapport à ladite zone, l'ensemble des signaux ultrasonores formant une image ultrasonore de la zone,
- Choisir une dimension de l'image ultrasonore et pour chaque signal correspondant à un vecteur de l'image selon la dimension choisie, appliquer un premier filtre prédéterminé au signal, le filtre étant configuré de sorte à transformer un premier signal comprenant deux extrema de même signe en un second signal comprenant un extremum de signe opposé situé entre les deux extrema du premier signal,
- Sélectionner, sur l'ensemble des signaux, le signal pour lequel le résultat du filtre présente l'extremum de valeur absolue la plus élevée et relever l'abscisse de cet extremum,
- Déterminer le centre de l'objet à partir de l'abscisse relevée et de la vitesse du signal ultrasonore.

Selon un aspect particulier de l'invention, le filtre est appliqué à l'enveloppe du signal ultrasonore ou à la valeur absolue du signal ultrasonore.

Selon un aspect particulier de l'invention, le filtre est appliqué au signal sur une fenêtre glissante de taille prédéfinie en fonction de la taille du signal et/ou d'une information a priori sur la dimension de l'objet, le filtre étant défini sur au moins trois intervalles temporels consécutifs par trois fonctions respectives pondérées chacune par un coefficient, les coefficients associés à deux intervalles temporels consécutifs étant de signes opposés.

Selon un aspect particulier de l'invention, la dimension du deuxième intervalle temporel est choisie de sorte à être strictement inférieure au diamètre minimum de l'objet à caractériser.

Selon un aspect particulier de l'invention, le filtre est défini sur au moins deux intervalles temporels supplémentaires.

Selon un aspect particulier de l'invention, chacune des fonctions est prise parmi : une somme, une valeur maximale, une moyenne ou une combinaison de ces fonctions.

Dans une variante de réalisation, la méthode selon l'invention comprend en outre les étapes de :
- Sélectionner le signal ultrasonore acquis pour lequel le centre de l'objet a été déterminé,
- Appliquer un seuil prédéterminé audit signal ultrasonore sélectionné,
- Détecter au moins deux extrema dudit signal supérieurs au seuil,
- Sélectionner le couple d'extrema, comprenant un premier extremum et un deuxième extremum, les plus proches du centre de l'objet et situés de part et d'autre du centre de l'objet, relever leurs abscisses temporelles respectives et en déduire le diamètre interne de l'objet à partir de la différence entre les deux abscisses et la vitesse du signal ultrasonore.

Dans une variante de réalisation, la méthode selon l'invention comprend les étapes de :
- Sélectionner un troisième extremum supérieur au seuil et situé immédiatement avant le premier extremum,
- Sélectionner un quatrième extremum supérieur au seuil et situé immédiatement après le second extremum,
- Relever les abscisses temporelles du troisième extremum et du quatrième extremum et en déduire le diamètre externe de l'objet à partir de la différence entre les deux abscisses et la vitesse du signal ultrasonore.

Selon un aspect particulier de l'invention, l'objet est un vaisseau sanguin, par exemple une artère.

Selon un aspect particulier de l'invention, l'étape d'acquérir plusieurs signaux ultrasonores comporte les sous étapes de :
- Positionner un transducteur comprenant plusieurs éléments alignés, sur une zone de la peau de sorte à imager une section transversale du vaisseau sanguin,
- Réaliser plusieurs acquisitions ultrasonores successives à partir de différents points d'émission situés sur l'axe d'alignement des éléments, chaque émission ultrasonore étant réalisée dans une direction sensiblement perpendiculaire à l'axe d'alignement.

L'invention a aussi pour objet un dispositif d'imagerie ultrasonore comprenant un transducteur ultrasonore et une unité de traitement configurée pour réaliser les étapes de la méthode selon l'invention.

D'autres caractéristiques et avantages de la présente invention apparaîtront mieux à la lecture de la description qui suit en relation aux dessins annexés suivants.
[Fig. 1] représente un schéma d'une sonde multiéléments ultrasonore apte à réaliser une séquence d'acquisition de signaux ultrasonores pour imager un vaisseau sanguin,
[Fig. 2] représente un organigramme décrivant les étapes de mise en œuvre d'une méthode de détection du centre d'un vaisseau sanguin selon un mode de réalisation de l'invention,
[Fig. 3a] représente une image ultrasonore d'une zone d'un plan transversal comprenant une artère radiale,
[Fig. 3b] représente l'image de la figure 3a filtrée au moyen d'un premier filtre défini selon l'invention,
[Fig. 3c] représente une l'image de la figure 3b filtrée au moyen d'un second filtre défini selon l'invention, de sorte à permettre la détection de l'artère radiale,
[Fig. 4a] représente un diagramme temporel d'un signal ultrasonore acquis au moyen de la sonde de la figure 1,
[Fig. 4b] représente un exemple de filtre destiné à être appliqué au signal de la figure 4a,
[Fig. 4c] représente le résultat de l'application du filtre de la figure 4b au signal de la figure 4a
[Fig. 4d] représente le résultat d'une étape de filtrage supplémentaire appliquée au signal de la figure 4c,
[Fig. 5] représente un organigramme décrivant les étapes de mise en œuvre d'une méthode de détermination du diamètre d'un vaisseau sanguin selon un mode de réalisation de l'invention,
[Fig. 6] représente un exemple de signal ultrasonore exploité pour déterminer le diamètre d'un vaisseau sanguin selon la méthode de la figure 5,

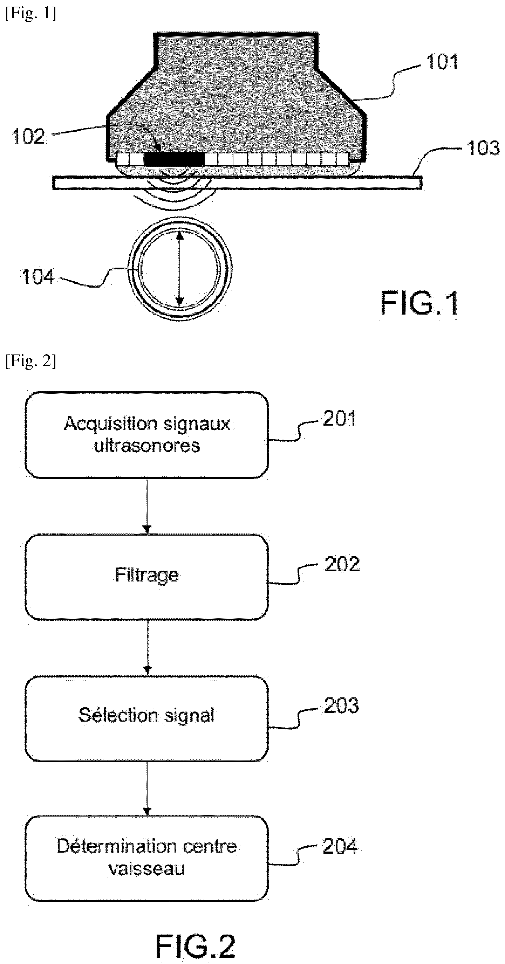

La figure 2 représente, sur un organigramme, les étapes principales de mise en œuvre d'une méthode de détermination du centre d'un vaisseau sanguin, par exemple une artère radiale, selon un mode de réalisation de l'invention.

La méthode débute à l'étape 201 par une acquisition de signaux ultrasonores dans un plan transversal de l'artère ou du vaisseau.

Cette acquisition est réalisée au moyen d'une sonde représentée à la figure 1.

La sonde d'acquisition est avantageusement une sonde linéaire multiéléments 101, c'est-à-dire qui comporte plusieurs éléments ultrasonores 102, par exemple des éléments piézo-électriques, alignés sur une rangée. Alternativement, une sonde en deux dimensions, c'est-à-dire comprenant une matrice d'éléments ultrasonores peut aussi être utilisée.

Dans l'exemple de la figure 1, la sonde 101 est positionnée au contact de la peau 103 de sorte que la rangée d'éléments 102 se trouve sensiblement dans un plan transversal d'une artère 104 à imager. La sonde 101 n'est pas nécessairement centrée sur l'artère 104, il suffit que l'artère soit couverte en émission et en réception par le faisceau ultrasonore généré par la sonde de sorte à obtenir une empreinte transversale de l'artère.

L'acquisition 101 est réalisée au moyen d'un balayage successif au cours duquel, à chaque étape, un faisceau ultrasonore est émis par un groupe d'éléments 102 comprenant au moins un élément, dans une direction perpendiculaire à l'axe de l'alignement des éléments. Les mêmes éléments sont actifs en réception pour générer un signal ultrasonore correspondant à une durée d'acquisition prédéfinie et à un axe sensiblement perpendiculaire au groupe d'éléments 102.

Cette étape permet d'acquérir un signal ultrasonore. Elle est ensuite itérée en décalant, selon une fenêtre glissante, les éléments ultrasonores actifs, d'un élément puis en réalisant une nouvelle acquisition. Ainsi, en réalisant plusieurs acquisitions successives au moyen d'un groupe d'éléments de taille fixe qui balaye l'ensemble des éléments de la sonde, on obtient plusieurs signaux ultrasonores qui forment ensemble une image ultrasonore d'un plan transversal de l'artère 104.

Cette acquisition permet d'obtenir en deux dimensions une empreinte ultrasonore transversale de l'artère radiale ciblée ainsi que d'éventuels autres objets anatomiques présents autour tels que des veines.

Sans sortir du cadre de l'invention, d'autres méthodes d'acquisition ultrasonores peuvent être envisagées dans la mesure où elles permettent l'obtention d'une image 2D dudit plan transversal. Sur l'image obtenue, qui correspond à une matrice d'échantillons de signaux, la position verticale représente la profondeur, c'est-à-dire la distance entre la sonde et un élément anatomique situé sous la sonde. La position horizontale représente un point sur la peau 103 correspondant au point d'émission du faisceau ultrasonore.

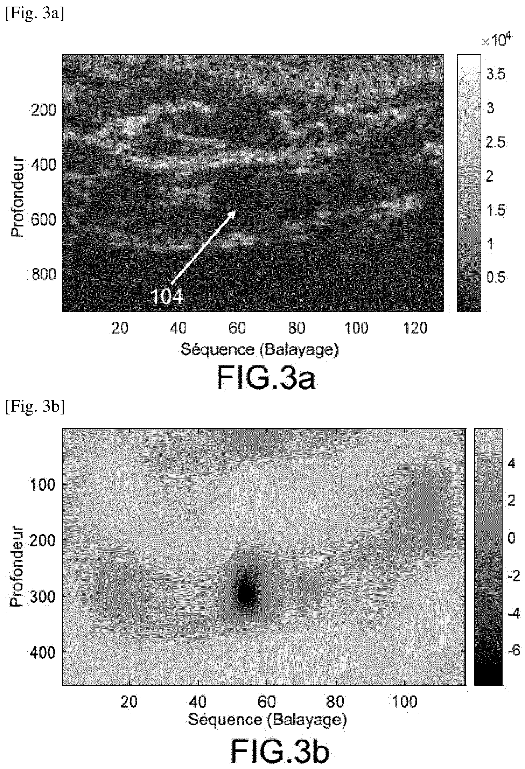

La figure 3a représente un exemple d'image ultrasonore obtenue avec identification de l'artère radiale 104. On peut voir que sur cette image, l'empreinte de l'artère 104 est difficilement détectable car elle est noyée dans le bruit et polluée par les empreintes d'autres objets anatomiques.

Un objectif de l'invention est d'identifier le point sur la peau situé à l'aplomb du centre de l'artère afin de déterminer la mesure ultrasonore qui correspond à un trajet passant par ce centre.

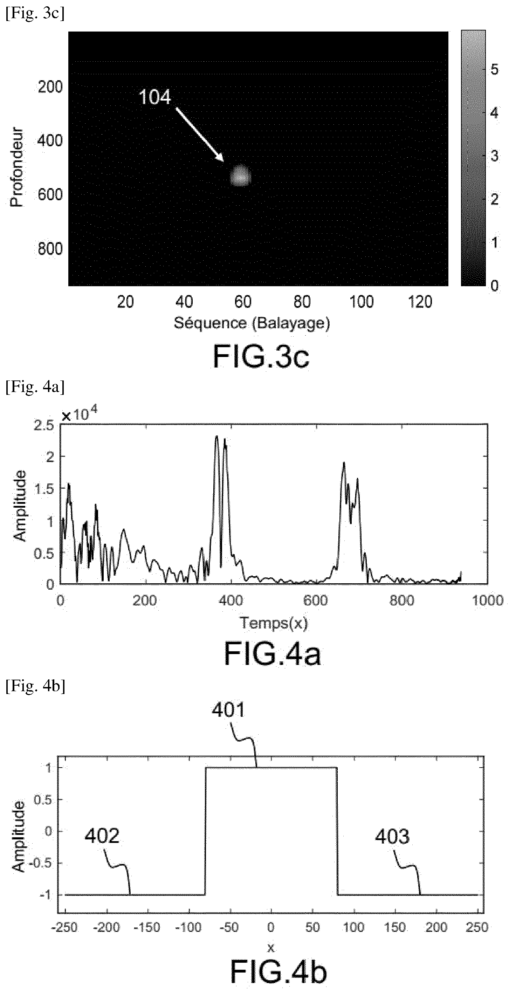

La figure 4a représente un exemple d'un signal ultrasonore acquis lors d'une acquisition simple. Le signal de la figure 4a correspond à une colonne de la matrice de la figure 3a. En effet, chaque colonne de cette matrice correspond à une acquisition à partir d'un point de la surface de la peau. Plus précisément, le signal de la figure 4a correspond à l'enveloppe ou la valeur absolue du signal ultrasonore acquis.

A l'étape 202, on applique ensuite, pour chacun des signaux acquis (chaque colonne de la matrice 2D), une ou plusieurs étapes de filtrage adapté spécifiquement pour identifier le centre de l'artère.

Comme cela peut être vu sur la figure 4a, une acquisition dans le plan transversal de l'artère est caractérisée par deux pics ou groupes de pics d'amplitude qui correspondent aux échos sur les parois de l'artère. Entre ces deux pics, se situe l'intérieur de l'artère.

Un premier filtre est appliqué au signal de la figure 4a. Ce filtre est défini de sorte à transformer le signal de la figure 4a qui comprend deux extrema de même signe en un autre signal qui comprend un extremum de signe opposé situé sensiblement à mi-distance des deux extrema du premier signal.

Un exemple de filtre est représenté à la figure 4b. Ce filtre correspond à un créneau temporel. Il présente une taille totale supérieure au diamètre de l'artère que l'on souhaite détecter et est composé de trois parties successives. La partie centrale 401 est fixée à une valeur positive, par exemple égale à 1, sur une durée correspondant à une distance inférieure au diamètre interne de l'artère.

Les deux autres parties 402, 403 sont fixées à une valeur négative, par exemple égale à -1.

Plus généralement il est possible de remplacer la valeur 1 par une autre valeur positive et la valeur -1 par une autre valeur négative.

En appliquant une convolution de ce filtre avec le signal acquis, les parties extérieures du filtre 402, 403 contribuent négativement aux résultats de convolution, tandis que la zone centrale 401 contribue positivement aux résultats de convolution.

Lorsque ce filtre est appliqué sur le signal acquis au niveau de la zone correspondant à l'artère, les pics d'amplitude correspondant aux parois de l'artère vont être alignés avec les zones extérieures du filtre 402, 403, leur contribution sera donc sommée négativement. A l'inverse, lorsque la partie centrale du filtre ne coïncide pas avec la zone centrale entre les deux pics d'amplitude mais coïncide avec l'un des pics d'amplitude, alors le résultat de la convolution avec le filtre sera une valeur positive.

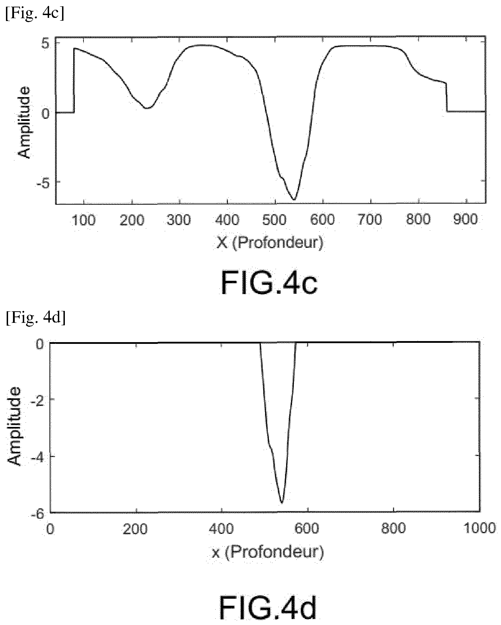

La figure 4c montre le résultat d'application du filtre de la figure 4b au signal de la figure 4a. On peut voir que le signal filtré obtenu présente des amplitudes positives partout sauf dans la zone correspondant à l'intérieur de l'artère.

La figure 4d montre le résultat final obtenu en mettant à zéro toutes les valeurs positives. L'extremum du signal de la figure 4d correspond à un point de la zone intérieure de l'artère pour lequel il n'y a que des contributions négatives, c'est-à-dire une situation où la zone centrale 401 du filtre est appliquée entre les deux pics d'amplitude correspondant aux parois de l'artère.

Ainsi, en analysant les signaux filtrés du type de la figure 4d pour l'ensemble des acquisitions, il est possible de repérer le centre de l'artère.

Le filtre décrit à la figure 4b est un exemple non limitatif et peut être adapté à la situation que l'on souhaite analyser. En particulier, les dimensions des trois parties 401, 402, 403 du filtre peuvent être adaptées selon les dimensions maximales des vaisseaux sanguins que l'on souhaite détecter.

En particulier, la dimension de la partie centrale 402 peut être choisie strictement inférieure au diamètre minimum d'un vaisseau que l'on souhaite caractériser. Alternativement, cette dimension peut être choisie de l'ordre du diamètre moyen des vaisseaux à caractériser. Le choix du dimensionnement dépend également du type de fonction de base appliquée par le filtre (maximum, minimum, moyenne ou somme notamment).

D'autres variantes de filtre peuvent être envisagées telles que décrites à présent.

Si on note *S* l'enveloppe ou la valeur absolue du signal ultrasonore acquis, ce signal étant composé de Nₓ échantillons:
$S = \left\{\left.i\left(x\right)\right|1\leq x\leq {N}_{x}\right\} ,$ où i(x) est l'intensité ultrasonore (amplitude) au point x du signal *S*.

On note ROI*ᵣ =*{i(*aᵣ*), *i*(*aᵣ* + 1), i(*aᵣ* + 2)..., i(*bᵣ*), |*aᵣ* ≤ x ≤ *bᵣ ;* (*aᵣ, bᵣ*) ∈ *S*}, où, ROI*ᵣ* est une région d'intérêt *r* représentée par l'ensemble de points du signal *S* situés dans l'intervalle [*aᵣ* , *bᵣ*]*.* Le filtre est défini par l'application de fonctions à plusieurs régions d'intérêt. Dans l'exemple de la figure 4b le nombre de régions d'intérêt est égal à trois mais il peut être supérieur à trois comme cela sera décrit plus loin.

La forme générale du filtre appliqué au signal est donnée par :
$F = {\sum }_{r = 1}^{{N}_{r}} {α}_{r} {f}^{r} \left({ROI}_{r}\right) ,$ où *f^{r}* est une fonction à valeurs strictement positives appliquée au signal, tel que par exemple, le maximum, la somme ou la moyenne sur l'ensemble des points appartenant à la région *ROIᵣ. αᵣ* est un coefficient de pondération prédéfini. Dans l'exemple de la figure 4b, il y a trois zones d'intérêts, donc Nᵣ=3 et les fonctions *f^{r}* sont toutes la fonction somme avec des coefficients égaux à -1 pour les zones d'intérêts extérieures et +1 pour la zone d'intérêt centrale du filtre.

Plus généralement, les coefficients peuvent avoir une valeur absolue différente de 1, le filtre F s'écrit alors :
$F = {α}_{1} {f}^{1} \left({ROI}_{paroiProximale}\right) + {α}_{2} {f}^{2} \left({ROI}_{ParoiDistale}\right) - {α}_{3} {f}^{3} \left({ROI}_{Vall é e}\right)$

La zone d'intérêt ROI_{vallée} correspond à la zone centrale du filtre et les zones d'intérêts ROI_{paroiProximale} et ROI_{paroiDistale} correspondent aux zones extérieures du filtre.

Pour l'exemple de la figure 4b, le filtre peut s'exprimer comme :
$F = somme \left({ROI}_{Vall é e}\right) - somme \left({ROI}_{paroiProximale}\right) - somme \left({ROI}_{ParoiDistale}\right)$

D'autre formes de filtre peuvent être considérées, soit calculées directement, soit calculées localement sur les points détectés par une première étape de filtrage.

En particulier, des filtres composés de cinq zones d'intérêts peuvent être considérés afin de prendre en compte les parois externes et internes d'un vaisseau. En effet la paroi externe et la paroi interne sont séparées d'une couche médiane non échogène. Le signal acquis peut donc présenter, pour chacune des parois proximale et distale, deux pics d'amplitude séparés par une zone proche de zéro, les deux pics correspondant aux parois externes et internes.$F = \begin{matrix}+ {α}_{1} {f}^{1} \left({ROI}_{paroiProximale}\right) - {α}_{4} {f}^{4} \left({ROI}_{CoucheMedianProximale}\right) \\ + {α}_{2} {f}^{2} \left({ROI}_{ParoiDistale}\right) - {α}_{5} {f}^{5} \left({ROI}_{CoucheMedianDistale}\right) \\ - {α}_{3} {f}^{3} \left({ROI}_{Vall é e}\right)\end{matrix}$

Un exemple particulier d'un filtre comprenant cinq zones d'intérêts est :
$F = \begin{matrix}+ Max \left({R}_{paroiProximale}\right) - moy \left({R}_{CoucheMedianProximale}\right) \\ + Max \left({R}_{ParoiDistale}\right) - moy \left({R}_{CoucheMedianDistale}\right) \\ - moy \left({R}_{Vall é e}\right)\end{matrix}$

Max est la valeur maximale du signal sur la zone d'intérêt et moy() est la moyenne du signal sur la zone d'intérêt.

Un autre exemple de réalisation consiste à normaliser le filtre initial par la moyenne de la région d'intérêt centrale ou vallée.$F ′ = \frac{F}{moy \left({ROI}_{Vall é e}\right)}$

Dans une autre variante de réalisation, en cas de présence d'objets anatomiques similaires, il est possible d'augmenter le filtre pour considérer des propriétés extrinsèques de l'objet ciblé, par exemple si l'artère radiale a une veine de chaque côté, la forme suivante permettra de le distinguer :
$F " = F - {α}_{6} {f}^{6} \left({ROI}_{veineGauche}\right) - {α}_{7} {f}^{7} \left({ROI}_{veineDroite}\right)$

Dans ce mode de réalisation, le filtre est composé de sept zones.

De façon générale, le filtre est défini sur au moins trois intervalles temporels consécutifs par trois fonctions respectives pondérées chacune par un coefficient, les coefficients associés au premier intervalle temporel et au troisième intervalle temporel étant de mêmes signes et le coefficient associé au deuxième intervalle temporel étant de signe opposé.

Lorsque le filtre est défini sur cinq intervalles temporels, il est alors défini sur au moins un premier intervalle temporel supplémentaire situé avant le premier intervalle temporel et un second intervalle temporel supplémentaire situé après le troisième intervalle temporel, les coefficients associés aux deux intervalles temporels supplémentaires étant de même signe que le coefficient associé au deuxième intervalle temporel.

Autrement dit, les coefficients du filtre correspondant à deux intervalles consécutifs sont de signes opposés.

De façon générale, l'alternance des signes des coefficients du filtre sur deux intervalles consécutifs permet d'obtenir un signal filtré qui présente un extrema lorsque le filtre coïncide temporellement avec le centre de l'intervalle délimité par deux pics d'amplitude du signal initial, ces pics correspondant aux échos du signal sur les parois du vaisseau sanguin.

La mise en œuvre du filtre peut être réalisée en cascade, de façon itérative, par exemple en appliquant le filtre sur l'intégralité du signal lors d'une première itération puis en appliquant un autre filtre au résultat du premier filtrage, centré sur une zone temporelle réduite, lors des itérations suivantes.

L'étape de filtrage 202 est appliquée à tous les signaux ultrasonores acquis correspondant à tous les vecteurs colonnes de l'image ultrasonore de la figure 3a.

Alternativement ou en supplément, il est possible également d'appliquer les mêmes traitements à chaque vecteur ligne de l'image ultrasonore afin d'identifier le centre de l'artère selon la dimension parallèle à l'axe de la sonde linéaire.

La figure 3b représente le résultat de l'application du filtre de la figure 4b pour l'ensemble des signaux (équivalent à la figure 4c pour un signal).

La figure 3c représente le résultat final après mise à zéro des valeurs positives. On peut identifier précisément l'artère 104 sur l'image de la figure 3c.

Le résultat de chaque filtrage peut être stocké en mémoire et rafraichi incrémentalement en gardant à chaque fois l'optimum. Il est possible aussi de stocker partiellement le résultat optimal de chaque signal, ou de stocker entièrement l'ensemble des résultats de filtrage, selon les ressources disponibles puis, à l'étape 203 de la méthode, on recherche ensuite, parmi tous les signaux filtrés, celui qui comprend l'extremum d'amplitude (en valeur absolue) la plus élevée. Le signal correspondant sélectionné sera celui qui correspond au trajet qui passe par le centre de l'artère. En effet, ce signal doit avoir une zone de vallée (entre les deux parois de l'artère) la plus importante et donc générer la contribution négative la plus importante parmi l'ensemble des signaux. Cela est renforcé aussi par la paroi qui a souvent une amplitude maximale au centre.

A l'étape 204, on détermine enfin le centre du vaisseau sanguin en relevant l'abscisse de l'extremum mesurée sur le signal sélectionné.

Cette abscisse temporelle t est ensuite convertie en distance d à partir de la vitesse v du signal ultrasonore dans le milieu : $D = \frac{1}{2} V ⋅ t$

On décrit à présent un second mode de réalisation dans l'invention qui consiste à déterminer ensuite le diamètre du vaisseau sanguin dont on a détecté le centre.

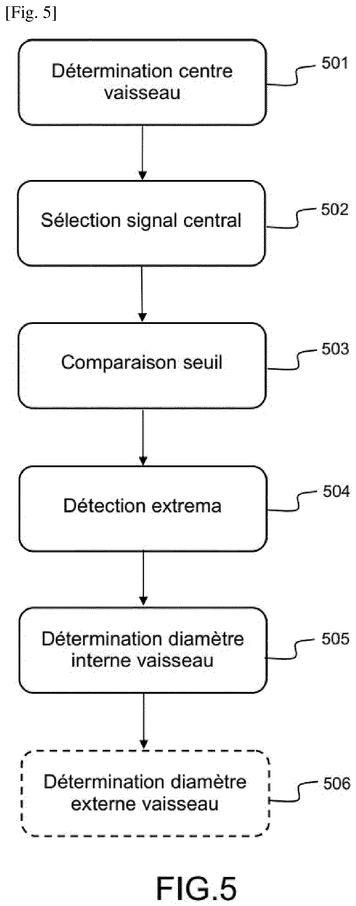

Ce second mode de réalisation est décrit à la figure 5.

Il débute à l'étape 501 par la détermination du centre du vaisseau au moyen de la méthode décrite précédemment à l'appui de la figure 2.

A l'étape 502, on sélectionne le signal précédemment sélectionné à l'étape 203 qui correspond au trajet ultrasonore qui passe par le centre du vaisseau.

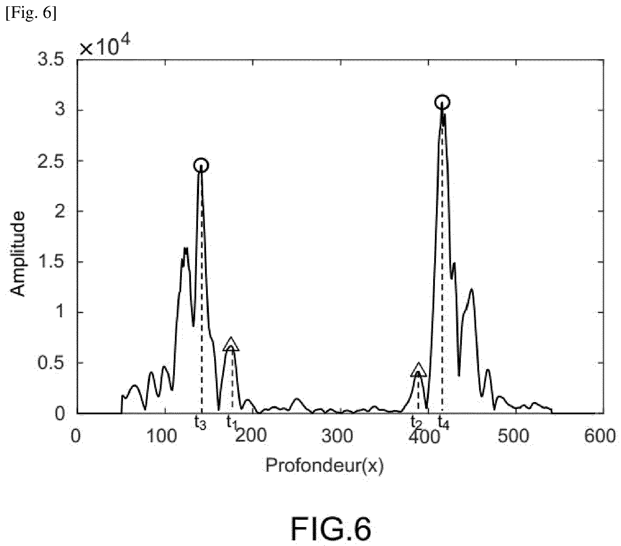

La figure 6 montre un exemple d'un tel signal sur lequel on a identifié plusieurs pics d'extremum.

A l'étape 503, on applique un seuil au signal afin de ne conserver que les points correspondants aux zones des extrema.

A l'étape 504, on détecte les deux points correspondant aux deux extrema les plus proches du centre du vaisseau déterminé à l'étape 501.

A l'étape 505, on en déduit le diamètre interne du vaisseau. En effet, les deux extrema détectés correspondent aux parois internes du vaisseau. Le diamètre interne du vaisseau est alors égal $à = \frac{1}{2} V ⋅ \left({t}_{2}-{t}_{1}\right)$, où t₂, t₁ sont les abscisses des deux extrema identifiés sur la figure 6.

Dans une étape optionnelle 506, on détermine en outre le diamètre externe du vaisseau à l'aide de la même relation $D = \frac{1}{2} V ⋅ \left({t}_{4}-{t}_{3}\right)$, où t₃, t₄ sont les abscisses de deux autres extrema dépassant le seuil de l'étape 503 et situés immédiatement après une zone de basse intensité qui présente une faible échogénéicité et qui est intermédiaire entre la paroi interne et la paroi externe de chacun des cotés proximal et distal. Autrement dit, les extrema détectés à l'étape 506 sont les extrema situés immédiatement de part et d'autre des premiers extrema détectés à l'étape 505 comme représenté sur la figure 6.

Les différentes abscisses des quatre extrema considérés sont identifiées sur la figure 6.

Comme expliqué précédemment, la méthode de la figure 5 peut aussi être appliquée sur la dimension horizontale de l'image ultrasonore, afin de déterminer le diamètre du vaisseau selon cette dimension.

Bien que l'invention ait été décrite préférentiellement pour des applications médicales consistant à caractériser un vaisseau sanguin et plus précisément une artère radiale, elle peut s'appliquer plus généralement pour toute application impliquant des images ultrasonores d'objets de forme tubulaires.

### Références

[1] J. H. Gagan et al., "Automated Segmentation of Common Carotid Artery in Ultrasound Images," in IEEE Access, vol. 10, pp. 58419-58430, 2022
[2] « System A feasibility study of a PMUT-based wearable sensor for the automatic monitoring of carotid artery parameters », 2021 IEEE International Ultrasonics Symposium (IUS)

## Revendications

1. Méthode de caractérisation d'un objet de forme tubulaire par imagerie ultrasonore, la méthode comprenant les étapes de :
- Acquérir (201), au moyen d'un transducteur ultrasonore, plusieurs signaux ultrasonores provenant de la réflexion d'un champ ultrasonore émis par le transducteur sur une zone d'intérêt dans un plan de coupe transversale de l'objet, pour différentes positions du transducteur par rapport à ladite zone, l'ensemble des signaux ultrasonores formant une image ultrasonore de la zone,
- Choisir une dimension de l'image ultrasonore et pour chaque signal correspondant à un vecteur de l'image selon la dimension choisie, appliquer (202) un premier filtre prédéterminé au signal, le filtre étant configuré de sorte à transformer un premier signal comprenant deux extrema de même signe en un second signal comprenant un extremum de signe opposé situé entre les deux extrema du premier signal,
- Sélectionner (203), sur l'ensemble des signaux, le signal pour lequel le résultat du filtre présente l'extremum de valeur absolue la plus élevée et relever l'abscisse de cet extremum,
- Déterminer (204) le centre de l'objet à partir de l'abscisse relevée et de la vitesse du signal ultrasonore.

2. Méthode de caractérisation d'un objet de forme tubulaire selon la revendication 1 dans laquelle le filtre est appliqué à l'enveloppe du signal ultrasonore ou à la valeur absolue du signal ultrasonore.

3. Méthode de caractérisation d'un objet de forme tubulaire selon la revendication 2 dans laquelle le filtre est appliqué au signal sur une fenêtre glissante de taille prédéfinie en fonction de la taille du signal et/ou d'une information a priori sur la dimension de l'objet, le filtre étant défini sur au moins trois intervalles temporels consécutifs par trois fonctions respectives pondérées chacune par un coefficient, les coefficients associés à deux intervalles temporels consécutifs étant de signes opposés.

4. Méthode de caractérisation d'un objet de forme tubulaire selon la revendication 3 dans laquelle, la dimension du deuxième intervalle temporel est choisie de sorte à être strictement inférieure au diamètre minimum de l'objet à caractériser.

5. Méthode de caractérisation d'un objet de forme tubulaire selon l'une quelconque des revendications 3 ou 4 dans laquelle le filtre est défini sur au moins deux intervalles temporels supplémentaires.

6. Méthode de caractérisation d'un objet de forme tubulaire selon l'une quelconque des revendications 3 à 5 dans laquelle chacune des fonctions est prise parmi : une somme, une valeur maximale, une moyenne ou une combinaison de ces fonctions.

7. Méthode de caractérisation d'un objet de forme tubulaire selon l'une quelconque des revendications précédentes comprenant en outre les étapes de :
- Sélectionner (502) le signal ultrasonore acquis pour lequel le centre de l'objet a été déterminé,
- Appliquer (503) un seuil prédéterminé audit signal ultrasonore sélectionné,
- Détecter (504) au moins deux extrema dudit signal supérieurs au seuil,
- Sélectionner le couple d'extrema, comprenant un premier extremum et un deuxième extremum, les plus proches du centre de l'objet et situés de part et d'autre du centre de l'objet, relever leurs abscisses temporelles respectives et en déduire (505) le diamètre interne de l'objet à partir de la différence entre les deux abscisses et la vitesse du signal ultrasonore.

8. Méthode de caractérisation d'un objet de forme tubulaire selon la revendication 7 comprenant les étapes de :
- Sélectionner un troisième extremum supérieur au seuil et situé immédiatement avant le premier extremum,
- Sélectionner un quatrième extremum supérieur au seuil et situé immédiatement après le second extremum,
- Relever les abscisses temporelles du troisième extremum et du quatrième extremum et en déduire (506) le diamètre externe de l'objet à partir de la différence entre les deux abscisses et la vitesse du signal ultrasonore.

9. Méthode de caractérisation d'un objet de forme tubulaire selon l'une quelconque des revendications précédentes dans lequel l'objet est un vaisseau sanguin, par exemple une artère.

10. Méthode de caractérisation d'un vaisseau sanguin selon la revendication 9 dans laquelle l'étape d'acquérir (201) plusieurs signaux ultrasonores comporte les sous étapes de :
- Positionner un transducteur comprenant plusieurs éléments alignés, sur une zone de la peau de sorte à imager une section transversale du vaisseau sanguin,
- Réaliser plusieurs acquisitions ultrasonores successives à partir de différents points d'émission situés sur l'axe d'alignement des éléments, chaque émission ultrasonore étant réalisée dans une direction sensiblement perpendiculaire à l'axe d'alignement.

11. Dispositif d'imagerie ultrasonore comprenant un transducteur ultrasonore (101) et une unité de traitement configurée pour réaliser les étapes de la méthode selon l'une quelconque des revendications précédentes.

## Patentansprüche

1. Verfahren zur Charakterisierung eines Objekts röhrenförmiger Form durch Ultraschallbildgebung, wobei das Verfahren die Folgenden Schritte umfasst:
- Erfassen (201), mittels eines Ultraschallwandlers, mehrerer Ultraschallsignale, die aus der Reflexion eines Ultraschallfeldes stammen, das von dem Wandler auf einen Bereich von Interesse in einer Querschnittsebene des Objekts für unterschiedliche Positionen des Wandlers in Bezug auf den Bereich emittiert wurde, wobei die Gesamtheit der Ultraschallsignale ein Ultraschallbild des Bereichs bilden,
- Wählen einer Dimension des Ultraschallbildes, und für jedes Signal, das einem Vektor des Bilds gemäß der gewählten Dimension entspricht, Anwenden (202) eines ersten vorbestimmten Filters auf das Signal, wobei der Filter derart konfiguriert ist, um ein erstes Signal, das zwei Extremwerte des gleichen Vorzeichens umfasst, in ein zweites Signal zu transformieren, das einen Extremwert des entgegengesetzten Vorzeichens zwischen den zwei Extremwerten des ersten Signal umfasst,
- Auswählen (203), aus der Gesamtheit der Signale, des Signals, für das das Ergebnis des Filters den Extremwert mit dem höchsten Absolutwert aufweist und Feststellen der Abszisse dieses Extremwerts,
- Bestimmen (204) des Mittelpunkts des Objekts ausgehend von der festgestellten Abszisse und der Geschwindigkeit des Ultraschallsignals.

2. Verfahren zur Charakterisierung eines Objekts röhrenförmiger Form nach Anspruch 1, wobei der Filter auf die Hüllkurve des Ultraschallsignals oder auf den Absolutwert des Ultraschallsignals angewendet wird.

3. Verfahren zur Charakterisierung eines Objekts röhrenförmiger Form nach Anspruch 2, wobei der Filter über ein Gleitfenster vordefinierter Größe in Funktion der Größe des Signals und/oder von a priori Informationen über die Dimension des Objekts auf das Signal angewendet wird, wobei der Filter über mindestens drei aufeinanderfolgende Zeitintervalle durch drei Funktionen, die jeweils durch einen Koeffizienten gewichtet sind, definiert ist, wobei die mit zwei aufeinanderfolgenden Zeitintervallen assoziierten Koeffizienten von entgegengesetzten Vorzeichen sind.

4. Verfahren zur Charakterisierung eines Objekts röhrenförmiger Form nach Anspruch 3, wobei die Dimension des zweiten Zeitintervalls derart gewählt wird, um strikt kleiner zu sein als der minimale Durchmesser des zu charakterisierenden Objekts.

5. Verfahren zur Charakterisierung eines Objekts röhrenförmiger Form nach einem der Ansprüche 3 oder 4, wobei der Filter über mindestens zwei zusätzliche Zeitintervalle definiert ist.

6. Verfahren zur Charakterisierung eines Objekts röhrenförmiger Form nach einem der Ansprüche 3 bis 5, wobei jede der Funktionen genommen wird aus: einer Summe, einem Maximalwert, einem Mittelwert oder einer Kombination aus diesen Funktionen.

7. Verfahren zur Charakterisierung eines Objekts röhrenförmiger Form nach einem der vorstehenden Ansprüche, weiter umfassend die folgenden Schritte:
- Auswählen (502) des erfassten Ultraschallsignals, für das der Mittelpunkt des Objekts bestimmt worden ist,
- Anwenden (503) eines vorbestimmten Schwellenwerts auf das ausgewählte Ultraschallsignal,
- Detektieren (504) von mindestens zwei Extremwerten des Signals oberhalb des Schwellenwerts,
- Auswählen des Paares von Extremwerten, das einen ersten Extremwert und einen zweiten Extremwert umfasst, die am nächsten zu dem Mittelpunkt des Objekts vorliegen und sich beiderseits des Mittelpunkts des Objekts befinden, Feststellen ihrer jeweiligen zeitlichen Abszissen und daraus Ableiten (505) des internen Durchmessers des Objekts ausgehend von dem Unterschied zwischen den zwei Abszissen und der Geschwindigkeit des Ultraschallsignals.

8. Verfahren zur Charakterisierung eines Objekts röhrenförmiger Form nach Anspruch 7, weiter umfassend die folgenden Schritte:
- Auswählen eines dritten Extremwerts oberhalb des Schwellenwerts und der sich unmittelbar vor dem ersten Extremwert befindet,
- Auswählen eines vierten Extremwerts oberhalb des Schwellenwerts und der sich unmittelbar nach dem zweiten Extremwert befindet,
- Feststellen der zeitlichen Abszissen des dritten Extremwerts und des vierten Extremwerts und daraus Ableiten (506) des externen Durchmessers des Objekts ausgehend von dem Unterschied zwischen den zwei Abszissen und der Geschwindigkeit des Ultraschallsignals.

9. Verfahren zur Charakterisierung eines Objekts röhrenförmiger Form nach einem der vorstehenden Ansprüche, wobei das Objekt ein Blutgefäß ist, zum Beispiel eine Arterie.

10. Verfahren zur Charakterisierung eines Blutgefäßes nach Anspruch 9, wobei der Schritt des Erfassens (201) mehrerer Ultraschallsignale die folgenden Teilschritte umfasst:
- Positionieren eines Wandlers, der mehrerer ausgerichtete Elemente umfasst, auf einen Bereich der Haut derart, um einen Querschnitt des Blutgefäßes abzubilden,
- Durchführen mehrerer Ultraschallerfassungen nacheinander ausgehend von unterschiedlichen Emissionspunkten, die sich auf der Ausrichtungsachse der Elemente befinden, wobei jede Ultraschallemission in einer Richtung im Wesentlichen senkrecht zu der Ausrichtungsachse durchgeführt wird.

11. Ultraschallbildgebungsvorrichtung, umfassend einen Ultraschallwandler (101) und eine Behandlungseinheit, die konfiguriert ist zum Durchführen der Schritte des Verfahrens nach einem der vorstehenden Ansprüche.

## Claims

1. Method for characterizing a tubular object by ultrasonic imaging, the method comprising the steps of:
- Acquiring (201), by means of an ultrasonic transducer, a plurality of ultrasonic signals originating from the reflection of an ultrasonic field transmitted by the transducer from a region of interest in a transverse cross-sectional plane of the object, for different positions of the transducer relative to said region, all of the ultrasonic signals forming an ultrasonic image of the region,
- Selecting a dimension of the ultrasonic image and, for each signal corresponding to a vector of the image along the selected dimension, applying (202) a predetermined first filter to the signal, the filter being configured so as to convert a first signal comprising two extrema of the same sign into a second signal comprising an extremum of opposite sign situated between the two extrema of the first signal,
- Selecting (203), from all of the signals, the signal for which the result of the filter contains the extremum having the highest absolute value, and recording the abscissa of said extremum,
- Determining (204) the center of the object from the recorded abscissa and the propagation velocity of the ultrasonic signal.

2. Method for characterizing a tubular object according to claim 1, wherein the filter is applied to the envelope of the ultrasonic signal or to the absolute value of the ultrasonic signal.

3. Method for characterizing a tubular object according to claim 2, wherein the filter is applied to the signal over a sliding window having a predetermined size determined based on the size of the signal and/or a priori information regarding the dimension of the object, the filter being defined over at least three consecutive time intervals by three respective functions, each weighted by a coefficient, the coefficients associated with two consecutive time intervals having opposite signs.

4. Method for characterizing a tubular object according to claim 3, wherein the duration of the second time interval is selected to be strictly less than the minimum diameter of the object to be characterized.

5. Method for characterizing a tubular object according to either claim 3 or claim 4, wherein the filter is defined over at least two additional time intervals.

6. Method for characterizing a tubular object according to any one of claims 3 to 5, wherein each of the functions is selected from the group consisting of: a sum, a maximum value, an average, or a combination thereof.

7. Method for characterizing a tubular object according to any one of the preceding claims, further comprising the steps of:
- Selecting (502) the acquired ultrasonic signal for which the center of the object has been determined,
- Applying (503) a predetermined threshold to the selected ultrasonic signal,
- Detecting (504) at least two extrema of the signal that are greater than the threshold,
- Selecting the pair of extrema, comprising a first extremum and a second extremum, that are closest to the center of the object and located on opposite sides of the center of the object, determining their respective temporal abscissas, and deducing (505) the internal diameter of the object from the difference between the two abscissas and the propagation velocity of the ultrasonic signal.

8. Method for characterizing a tubular object according to claim 7, comprising the steps of:
- Selecting a third extremum greater than the threshold and located immediately before the first extremum,
- Selecting a fourth extremum greater than the threshold and located immediately after the second extremum,
- Determining the respective temporal abscissas of the third extremum and the fourth extremum, and deducing (506) the external diameter of the object from the difference between the two abscissas and the propagation velocity of the ultrasonic signal.

9. Method for characterizing a tubular object according to any one of the preceding claims, wherein the object is a blood vessel, for example, an artery.

10. Method for characterizing a blood vessel according to claim 9, wherein the step of acquiring (201) a plurality of ultrasonic signals comprises the substeps of:
- Positioning a transducer comprising a plurality of aligned elements on an area of the skin so as to image a cross section of the blood vessel,
- Performing a plurality of successive ultrasonic acquisitions from different transmission points located on the alignment axis of the elements, each ultrasonic transmission being performed in a direction substantially perpendicular to the alignment axis.

11. Ultrasonic imaging device comprising an ultrasonic transducer (101) and a processing unit configured to perform the steps of the method according to any one of the preceding claims.
